(19) Europäisches Patentamt
European Patent Office
Office européen des brevets

(11) Veröffentlichungsnummer: **0 004 532**
**B1**

(12) # EUROPÄISCHE PATENTSCHRIFT

(45) Veröffentlichungstag der Patentschrift: **14.10.81**

(51) Int. Cl.³: **C 07 C 93/14,** C 07 D 209/04, C 07 D 209/82, C 07 C 103/38, C 07 C 121/75

(21) Anmeldenummer: **79100096.1**

(22) Anmeldetag: **12.01.79**

(54) **1-Aryloxy-3-nitratoalkylamino-2-propanole, Verfahren zu ihrer Herstellung und ihre pharmazeutischen Zubereitungen.**

(30) Priorität: **09.02.78 DE 2805404**

(43) Veröffentlichungstag der Anmeldung: **17.10.79 Patentblatt 79/21**

(45) Bekanntmachung des Hinweises auf die Patenterteilung: **14.10.81 Patentblatt 81/41**

(84) Benannte Vertragsstaaten: **BE CH DE FR GB IT NL SE**

(56) Entgegenhaltungen:
**DE-A-1 543 701**
**GB-B-1 364 280**

(73) Patentinhaber: **Merck Patent Gesellschaft mit beschränkter Haftung, Frankfurter Strasse 250, D-6100 Darmstadt (DE)**

(72) Erfinder: **Sombroek, Johannes, Dr., Walbeweg 3, D-6100 Darmstadt (DE)**
Erfinder: **Becker, Karl-Heinz, Dr., Tannenweg 4, D-6110 Dieburg (DE)**
Erfinder: **Minck, Klaus-Otto, Dr., Büchestrasse 8, D-6105 Ober-Ramstadt (DE)**
Erfinder: **Enenkel, Hans-Joachim, Dr., Wingertsbergstrasse 5, D-6100 Darmstadt (DE)**

**EP 0 004 532 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 1-Aryloxy-3-nitratoalkylamino-2-propanole, Verfahren zu ihrer Herstellung und ihre pharmazeutischen Zubereitungen

Die Erfindung betrifft neue 1-Aryloxy-3-nitratoalkylamino-2-propanole der allgemeinen Formel I

$$Ar-O-CH_2CHOH-CH_2-NH-R \qquad I$$

worin

Ar Phenyl; ein- oder mehrfach durch Halogen, Cyan, Acylamino (worin die Acylgruppe 1–7 C-Atome besitzt), Alkanoyl mit 1–4 C-Atomen, Alkyl (worin die Kette durch 1 oder 2 O-Atome oder durch ein S-Atom unterbrochen und/oder worin eine Mehrfachbindung vorhanden sein kann) mit insgesamt 1–10 C- Atomen, Cycloalkyl mit 3–8 C-Atomen und/oder Cycloalkoxy mit 3–8 C-Atomen substituiertes Phenyl; Naphthyl; Indanyl; Indenyl; Tetralyl; Indolyl; ein- oder mehrfach durch Alkyl mit jeweils 1–4 C-Atomen substituiertes Indolyl; Carbazolyl; oder 1,2,3,4-Tetrahydro-2-oxo-chinolyl und

R Nitratoalkyl mit 2–10 C-Atomen bedeuten,

und ihre physiologisch unbedenklichen Säureadditionssalze.

Ähnliche Verbindungen sind aus der GB-B-1 364 280 und und der DE-A-1 543 701 bekannt. So sind dort Verbindungen beschrieben, die im wesentlichen der Formel I entsprechen, aber an Stelle des Restes R Hydroxyalkylgruppen tragen. Auch »Ester« dieser Verbindungen sind genannt; es wird jedoch an keiner Stelle dieser Druckschriften festgestellt, daß auch die Nitrate als »Ester« der dort beschriebenen Hydroxyalkylverbindungen in Betracht gezogen werden können, und über die im Vergleich zu denen der zugrundeliegenden Hydroxyalkylverbindungen besonders vorteilhaften und überraschenden Eigenschaften der Nitrate wird nichts ausgesagt.

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I gelöst.

Es wurde gefunden, daß diese Verbindungen bei guter Verträglichkeit sehr wertvolle pharmakologische Eigenschaften besitzen. Vor allem blockieren sie die adrenergen $\beta$-Rezeptoren und zeigen isoprenalin-antagonistische Wirkungen auf die Herzfrequenz, z. B. von Meerschweinchen, Katzen oder Hunden, feststellbar z. B. nach der Methodik, die in der DE-AS 14 93 564 näher beschrieben ist. Einige der Verbindungen zeigen eine kardioselektive Wirkung. Außerdem bewirken sie eine günstige periphere Vasodilatation. Weiterhin treten cholesterinspiegelsenkende und triglyceridspiegelsenkende Wirkungen auf, die nach den von Levine und Mitarbeitern (Automation in Analytical Chemistry, Technicon Symposium, 1967, Mediad, New York, Seite 25–28) bzw. von Noble und Campbell (Clin. Chem. 16 (1970), Seiten 166–170) beschriebenen Methoden an Ratten ermittelt werden können. Ferner zeigen die Produkte Wirkungen auf das Zentralnervensystem sowie thrombozytenaggregationshemmende, antiarrhythmische und lipolysehemmende Wirkungen, die ebenfalls nach hierfür geläufigen Methoden ermittelt werden können. Die Verbindungen zeigen somit ein sehr breites Wirkungsspektrum.

Die Verbindungen können dementsprechend als Arzneimittelwirkstoffe in der Human- und Veterinärmedizin eingesetzt werden, insbesondere bei der Prophylaxe und bei der Behandlung von Herz-, Kreislauf- und Gefäßerkrankungen, z. B. von Angina pectoris und von Herzrhythmusstörungen. Ferner können sie als Zwischenprodukte zur Herstellung weiterer Arzneimittelwirkstoffe verwendet werden.

Gegenstand der Erfindung sind die neuen Verbindungen der Formel I sowie ihre physiologisch unbedenklichen Salze.

Der Rest Ar kann beispielsweise eine unsubstituierte oder substituierte Phenylgruppe bedeuten. Im letzten Fall ist die Phenylgruppe vorzugsweise einfach (insbesondere in o-Stellung, weiterhin in p-Stellung, aber auch in m-Stellung) oder zweifach (insbesondere in 2,5-Stellung, aber auch z. B. in 2,3-, 2,4-, 3,4-oder 3,5-Stellung) substituiert; sie kann aber auch drei- (insbesondere in 3,4,5-Stellung, aber auch z. B. in 2,3,4-, 2,3,5- oder 2,4,5-Stellung), vier- (z. B. in 2,3,4,5-Stellung) oder fünffach substituiert sein. Als Substituenten an der Phenylgruppe kommen insbesondere in Frage: F, Cl, Br, J; CN; Acylamino, vorzugsweise Alkanoylamino mit 1–7, vorzugsweise 1–4 C-Atomen wie Formylamino, Acetylamino, ferner Propionylamino, Butyrylamino, Isobutyrylamino, Valerylamino, Caproylamino, Heptanoylamino, ferner auch Aroylamino wie Benzoylamino; Alkanoyl mit 1–7, vorzugsweise 1–4 C-Atomen, vorzugsweise Formyl, Acetyl oder Propionyl, ferner z. B. Butyryl, Isobutyryl, Valeryl, Caproyl, Heptanoyl; Alkyl mit 1–10, vorzugsweise 1–4 C-Atomen, vorzugsweise Methyl oder Äthyl, ferner z. B. n-Propyl, Isopropyl, n-Butyl, Isobutyl, sek.-Butyl, tert.-Butyl, Pentyl wie n-Pentyl, Hexyl wie n-Hexyl, Heptyl wie n-Heptyl, Octyl wie n-Octyl, Nonyl wie n-Nonyl oder Decyl wie n-Decyl; Alkoxy mit 1–10, vorzugsweise 1–4 C-Atomen, vorzugsweise Methoxy oder Äthoxy, ferner z. B. n-Propoxy, Isopropoxy, n-Butoxy, Isobutoxy, sek.-Butoxy, tert.-Butoxy, Pentyloxy, Hexyloxy, Heptyloxy, Octyloxy, Nonyloxy oder Decyloxy; Alkoxyalkyl mit bis zu 10, vorzugsweise 2–6 C-Atomen, z. B. Alkoxymethyl wie Methoxymethyl, Alkoxyäthyl wie 1- oder 2-Methoxyäthyl, 1- oder 2-n-Butoxyäthyl, 1- oder 2-n-Octyloxyäthyl; Alkoxyalkoxyalkyl mit bis zu 10, vorzugsweise 4–7 C-Atomen, z. B. Alkoxyalkoxymethyl wie

2-Methoxyäthoxy-methyl, 2-Äthoxyäthoxy-methyl oder 2-Isopropoxyäthoxy-methyl, Alkoxyalkoxyäthyl wie 2-(2-Meth-oxyäthoxy)-äthyl oder 2-(2-Äthoxyäthoxy)-äthyl; Alkoxyalkoxy mit bis zu 10, vorzugsweise 3–6 C-Atomen, z. B. 2-Methoxyäthoxy, 2-Äthoxyäthoxy oder 2-n-Butoxyäthoxy; Alkylthio mit 1–10, vorzugsweise 1–4 C-Atomen, vorzugsweise Methylthio oder Äthylthio, ferner z. B. n-Propylthio, Isopropylthio, n-Butylthio, Isobutylthio, sek.-Butylthio, tert.-Butylthio, Pentylthio, Hexylthio, Heptylthio, Octylthio, Nonylthio oder Decylthio; Alkylthioalkyl mit bis zu 10, vorzugsweise 2–6 C-Atomen, z. B. Methylthiomethyl, 2-Methylthioäthyl oder 2-n-Butylthioäthyl; Alkenyl mit bis zu 10, vorzugsweise 2–4 C-Atomen, z. B. Vinyl, Allyl, Propenyl, Isopropenyl, Butenyl wie 1-Buten-1-, -2-, -3- oder -4-yl, 2-Buten-1-yl, 2-Buten-2-yl, Pentenyl, Hexenyl oder Decenyl; Alkenyloxy mit bis zu 10, vorzugsweise 2–4 C-Atomen, vorzugsweise Allyloxy, ferner z. B. Vinyloxy, Propenyloxy, Isopropenyloxy, Butenyloxy wie 1-Buten-1-, -2-, -3- oder -4-yloxy, 2-Buten-1-yloxy, 2-Buten-2-yloxy, Pentenyloxy, Hexenyloxy oder Decenyloxy; Alkenyloxyalkyl mit bis zu 10, vorzugsweise 3–6 C-Atomen, z. B. Allyloxymethyl; Alkinyl mit bis zu 10, vorzugsweise 2–4 C-Atomen, z. B. Äthinyl, 1-Propin-1-yl, Propargyl, Butinyl wie 2-Butin-1-yl, Pentinyl, Decinyl; Alkinyloxy mit bis zu 10, vorzugsweise 2–4 C-Atomen, vorzugsweise Propargyloxy, ferner z. B. Äthinyloxy, 1-Propin-1-yloxy, Butinyloxy wie 2-Butin-1-yloxy, Pentinyloxy oder Decinyloxy; Alkinyloxyalkyl mit bis zu 10, vorzugsweise 3–6 C-Atomen, z. B. Äthinyloxymethyl, Propargyloxymethyl oder 2-(2-Butin-1-yl-oxy)-äthyl; Cycloalkyl mit 3–8, vorzugsweise 5 oder 6 C-Atomen, vorzugsweise Cyclopentyl oder Cyclohexyl, ferner z. B. Cyclopropyl, Cyclobutyl, 1-, 2-oder 3-Methylcyclopentyl, 1-, 2-, 3- oder 4-Methylcyclohexyl, Cycloheptyl oder Cyclooctyl; Cycloalkoxy mit 3–8, vorzugsweise 5 oder 6 C-Atomen, vorzugsweise Cyclopentyloxy oder Cyclohexyloxy, ferner z. B. Cyclopropyloxy, Cyclobutyloxy, 1-, 2- oder 3-Methylcyclopentyloxy, 1-, 2-, 3- oder 4-Methylcyclohexyloxy, Cycloheptyloxy oder Cyclooctyloxy.

Der Rest Ar kann z. B. ferner bedeuten: 1- oder 2-Naphthyl; 1-, 2-, 3-, (bevorzugt) 4-, 5-, 6- oder 7-Indanyl; 1-, 2-, 3-, (bevorzugt) 4-, 5-, 6- oder 7-Indenyl; 1-, 2-, 3-, 4-, (bevorzugt) 5-, 6-, 7-oder 8-Tetralyl; (bevorzugt) 4-, 5-, 6- oder 7-Indolyl; Alkylindolyl, vorzugsweise Methylindolyl, z. B. 2-Methyl-4-indolyl oder 3-Methyl-4-indolyl, ferner z. B. 2-Äthyl-4-indolyl oder 3-Äthyl-4-indolyl; Dialkylindolyl, vorzugsweise Dimethylindolyl, z. B. 2,3-Dimethyl-4-indolyl, ferner z. B. 2-Methyl-3-äthyl-4-indolyl, 2-Äthyl-3-methyl-4-indolyl, 2,3-Diäthyl-4-indolyl; 1-, 2-, 3- oder (bevorzugt) 4-Carbazolyl; 1,2,3,4-Tetrahydro-2-oxo-3-, -4-, (bevorzugt) -5-, -6-, -7- oder -8-chinolyl.

Bevorzugte einzelne Reste Ar sind z. B. Phenyl; Halogenphenyl wie o-, m- oder p-Fluorphenyl, o-, m- oder p-Chlorphenyl, o-, m- oder p-Bromphenyl, o-, m- oder p-Jodphenyl; Dihalogenphenyl wie 2,3-, 2,4-, 2,5-, 3,4- oder 3,5-Dichlorphenyl; o-, m- oder p-Cyanphenyl; o-, m- oder p-Acetylaminophenyl; 2-Acetyl-4-butyrylaminophenyl; o-, m- oder p-Tolyl, o-, m- oder p-Äthylphenyl, o-, m- oder p-Isopropylphenyl, 2,3-, 2,4-, 2,5-, 3,4- oder 3,5-Dimethylphenyl; 2-Chlor-5-methylphenyl, 2-Methyl-5-chlorphenyl; o-, m- oder p-Methoxyphenyl, o-, m- oder p-Äthoxyphenyl, o-, m- oder p-2-Methoxyäthylphenyl, o-, m- oder p-(2-Isopropoxyäthoxymethyl)-phenyl, o-, m- oder p-Allylphenyl, o-, m- oder p-Allyloxyphenyl, o-, m- oder p-Propargyloxyphenyl, o-, m- oder p-Methylthiophenyl; o-, m- oder p-Cyclopropylphenyl; o-, m- oder p-Cyclopentylphenyl; o-, m- oder p-Cyclohexylphenyl; 1-Naphthyl; 4-Indanyl; 4-Indenyl; 5-Tetralyl; 4-Indolyl; 2-Methyl-4-indolyl; 2,3-Dimethyl-4-indolyl; 4-Carbazolyl; 1,2,3,4-Tetrahydro-2-oxo-5-chinolyl.

Der Rest R bedeutet eine geradkettige oder (vorzugsweise) verzweigte Nitratoalkylgruppe mit 2–10, vorzugsweise 2–6 C-Atomen. Besonders bevorzugt bedeutet R die Gruppe

$$-CL^1L^2-(CH_2)_m-CL^3L^4-O-NO_2,$$

worin $L^1$ bis $L^4$ jeweils H oder $CH_3$ und m 0–2 bedeuten. Einzelne bevorzugte Reste R sind

$$-CH_2CH_2-O-NO_2,\ -CH(CH_3)-CH(CH_2-O-NO_2,$$

$$-C(CH_3)_2-CH_2-O-NO_2,\ -CH(CH_3-CH(CH_3)-O-NO_2,$$

$$-CH_2-CH(CH_3)-O-NO_2,\ CH_2C(CH_3)_2O-NO_2,$$

$$CH_2CH_2CH_2-O-NO_2,\ -CH(CH_3)-CH_2-CH_2-O-NO_2,$$

$$-C(CH_3)_2-CH_2CH_2-O-NO_2,\ -C(CH_3)_2-CH_2-CH(CH_3)-O-NO_2,$$

$$-C(CH_3)_2-CH_2-C(CH_3)_2-O-NO_2,\ -CH(C_2H_5)-CH_2CH_2-O-NO_2,$$

$$-C(CH_3)(C_2C_5)-CH_2CH_2-O-NO_2,\ -C(C_2C_5)_2-CH_2CH_2-O-NO_2,$$

$$-CH(CH_3)-CH_2CH_2-CH_2-O-NO_2 \text{ und } -C(CH_3)_2-CH_2CH_2CH_2-O-NO_2.$$

Insbesondere sind folgende Reste R bevorzugt: 1-Methyl-3-nitratopropyl, 1,1-Dimethyl-3-nitratopropyl, ferner 1,1,3-Trimethyl-3-nitratopropyl, 1-Methyl-4-nitratobutyl und 1,1-Dimethyl-4-nitratobutyl.

Dementsprechend sind Gegenstand der Erfindung insbesondere diejenigen Verbindungen der Formel I, in denen mindestens einer der Reste Ar und R eine der vorstehend angegebenen bevorzugten Bedeutungen hat.

Einige bevorzugte Gruppen von Verbindungen können durch die folgenden Teilformeln Ia bis If ausgedrückt werden, die der Formel I entsprechen und worin die nicht näher bezeichneten Reste die bei der Formel I angegebene Bedeutung haben, worin jedoch

in Ia Ar Phenyl, Chlorphenyl, Dichlorphenyl, Chlormethyl-phenyl, Cyanphenyl, Acetylaminophenyl, Tolyl, Dimethylphenyl, Isopropylphenyl, Methoxyphenyl, Allylphenyl, Allyloxyphenyl, 2-Methoxyäthylphenyl, 2-Isopropoxyäthoxymethyl-phenyl, Methylthiophenyl, Cyclopropylphenyl, Cyclopentylphenyl, Cyclohexylphenyl, Naphthyl, Indenyl, Indolyl, Carbazolyl oder 1,2,3,4-Tetrahydro-2-oxo-chinolyl bedeutet;

in Ib Ar Phenyl, 2,5-Dichlorphenyl, 2-Chlor-5-methylphenyl, o-Cyanphenyl, p-Acetylaminophenyl, 2,3-Dimethylphenyl, o-Isopropylphenyl, o-Methoxyphenyl, o-Allylphenyl, o-Allyloxyphenyl, p-2-Isopropoxyäthoxymethyl-phenyl, o-Methylthiophenyl, o-Cyclohexylphenyl, 1-Naphthyl, 4-Indenyl, 4-Indolyl, 4-Carbazolyl oder 1,2,3,4-Tetrahydro-2-oxo-5-chinolyl bedeutet;

in Ic Ar o-Cyanphenyl, o-Isopropylphenyl, o-Methoxyphenyl, o-Allyloxyphenyl, 1-Naphthyl, 4-Indenyl, 4-Indolyl oder 4-Carbazolyl bedeutet;

in Id R 1-Methyl-3-nitratopropyl, 1,1-Dimethyl-3-nitratopropyl, 1,1,3-Trimethyl-3-nitratopropyl, 1-Methyl-4-nitratobutyl oder 1,1-Dimethyl-4-nitratobutyl bedeutet;

in Ie Ar o-Cyanphenyl, o-Isopropylphenyl, o-Methoxyphenyl, o-Allyloxyphenyl, 1-Naphthyl, 4-Indenyl, 4-Indolyl oder 4-Carbazolyl und
R 1-Methyl-3-nitratopropyl, 1,1-Dimethyl-3-nitratopropyl, 1,1,3-Trimethyl-3-nitratopropyl, 1-Methyl-4-nitratobutyl oder 1,1-Dimethyl-4-nitratobutyl bedeutet;

in If Ar o-Cyanphenyl, o-Isopropylphenyl, o-Methoxyphenyl, o-Allyloxyphenyl, 1-Naphthyl, 4-Indenyl, 4-Indolyl oder 4-Carbazolyl und
R 1-Methyl-3-nitratopropyl oder 1,1-Dimethyl-3-nitratopropyl bedeutet.

Die Verbindungen der Formel I besitzen mindestens ein asymmetrisches C-Atom und können in den Substituenten Ar und R weitere asymmetrische C-Atome enthalten. Sie können daher in racemischer oder in optisch-aktiver Form vorliegen. In der Regel fallen sie bei der Synthese als Racemate an.

Gegenstand der Erfindung ist ferner ein Verfahren zur Herstellung von 1-Aryloxy-3-nitratoalkylamino-2-propanolen der Formel I sowie von deren physiologisch unbedenklichen Säureadditionssalzen, das dadurch gekennzeichnet ist, daß man eine Verbindung der allgemeinen Formel II

$$Ar-O-CH_2-CHQ-CH_2Y \qquad II$$

mit einer Verbindung der allgemeinen Formel III

$$Z-R \qquad III$$

worin

der eine der Reste Y und Z $NH_2$,
der andere dieser Reste X,
Q OH oder zusammen mit X ein Sauerstoffatom,
X Hal, OH, eine funktionell abgewandelte OH-Gruppe oder (in II) zusammen mit Q ein Sauerstoffatom und
Hal Cl, Br oder J bedeuten und
Ar und R die oben angegebenen Bedeutungen haben,
oder ein Phenol der allgemeinen Formel IV

$$Ar-OH \qquad IV$$

worin

Ar die oben angegebene Bedeutung hat

mit einem Nitratoalkylamin der allgemeinen Formel V

$$X-CH_2-CHQ-CH_2-NH-R \qquad V$$

worin

R, Q und X die oben angegebenen Bedeutungen haben

umsetzt
oder daß man eine der allgemeinen Formel I entsprechende Verbindung, die jedoch zusätzlich an Stelle eines oder mehrerer H-Atome eine oder mehrere solvolytisch abspaltbare Gruppe(n) trägt, mit einem solvolysierenden Mittel behandelt,
oder daß man eine Verbindung der allgemeinen Formel VI

$$Ar{-}O{-}CH_2CHOH{-}CH_2{-}NH{-}E \qquad VI$$

worin

E eine Hydroxyalkylgruppe mit 2–10 C-Atomen bedeutet und
Ar die oben angegebene Bedeutung hat;

oder eines ihrer reaktionsfähigen Derivate mit Salpetersäure oder einem ihrer reaktionsfähigen Derivate verestert,
und daß man gegebenenfalls eine erhaltene Base der Formel I durch Behandeln mit einer Säure in eines ihrer physiologisch unbedenklichen Säureadditionssalze umwandelt.

In den Ausgangsstoffen der Formeln II, III und V kann der Rest X vorhanden sein. Dieser Rest ist vorzugsweise Cl oder Br, ferner auch J, OH oder eine funktionell abgewandelte OH-Gruppe. Unter funktionell abgewandelten OH-Gruppen sind hier insbesondere reaktionsfähig veresterte OH-Gruppen zu verstehen, z. B. Alkylsulfonyloxy mit vorzugsweise 1–6 C-Atomen wie Methansulfonyloxy oder Arylsulfonyloxy mit vorzugsweise 6–10 C-Atomen wie Benzolsulfonyloxy, p-Toluolsulfonyloxy oder 1- oder 2-Naphthalinsulfonyloxy.

Die Herstellung der Verbindungen der Formel I erfolgt im übrigen nach an sich bekannten Methoden, wie sie in der Literatur (z. B. in den Standard-Werken wie Houben–Weyl, Methoden der Organischen Chemie, Georg-Thieme-Verlag, Stuttgart; Organic Reactions, John Wiley und Sons, Inc., New York) beschrieben sind, und zwar unter Bedingungen, wie sie für die genannten Umsetzungen bekannt und geeignet sind. Dabei kann man auch von an sich bekannten, hier nicht näher erwähnten Varianten Gebrauch machen.

Die Ausgangsstoffe zur Herstellung der Verbindungen der Formel I sind teilweise bekannt, teilweise neu. Die neuen Ausgangsstoffe können nach an sich bekannten Verfahren, in der Regel in Analogie zu den bekannten Ausgangsstoffen, hergestellt werden.

Die Ausgangsstoffe können, falls erwünscht, auch in situ gebildet werden, derart, daß man sie aus dem Reaktionsgemisch nicht isoliert, sondern sofort weiter zu den Verbindungen der Formel I umsetzt.

Nachfolgend haben die Reste Ar, E, Hal, Q, R, X, Y und Z die bei den Formeln I bis V angegebenen Bedeutungen, sofern nicht ausdrücklich etwas anderes angegeben ist.

Im einzelnen sind die Ausgangsstoffe der Formel II in der Regel bekannt. Sie sind beispielsweise erhältlich durch Umsetzung der Phenole der Formel Ar–OH (IV) mit Verbindungen der Formel

$$X{-}CH_2{-}CHQ{-}CH_2Y$$

(z. B. Epichlorhydrin, Epibromhydrin). Primäre Amine der Formel II ($Y = NH_2$) können z. B. hergestellt werden durch Umsetzung von Epoxiden der Formel II (Q und Y bedeuten zusammen ein Sauerstoffatom) mit Ammoniak oder mit Benzylamin sowie anschließende hydrogenolytische Entfernung der Benzylgruppe.

Ausgangsstoffe der Formel III sind teils bekannt, teils neu. Die Amine der Formel III ($Z = NH_2$) können durch Veresterung der entsprechenden Aminoalkanole der Formel $H_2N{-}E$ mit Salpetersäure oder aus den entsprechenden Halogenverbindungen der Formel III (Z = Hal) durch Umsetzung mit Ammoniak erhalten werden. Verbindungen der Formeln II und III, in denen die Reste Y oder Z funktionell abgewandelte OH-Gruppen bedeuten, sind durch funktionelle Abwandlung der entsprechenden Alkohole zugänglich, z. B. durch Umsetzung mit Alkyl- oder Arylsulfonylhalogeniden in Gegenwart von Pyridin.

Die Phenole der Formel IV sind in der Regel bekannt. Sie können nach üblichen Phenolsynthesen erhalten werden. Nitratoalkylamine der Formel V können beispielsweise durch Umsetzung von Verbindungen der Formel $X{-}CH_2{-}CHQ{-}CH_2Y$ (vorzugsweise Epoxiden wie Epichlorhydrin) mit Aminen der Formel III ($Z = NH_2$) hergestellt werden.

Die Verbindungen der Formel I werden vorzugsweise durch Umsetzung der Verbindungen der Formel II mit den Verbindungen der Formel III hergestellt. Man kann einerseits Epoxide der Formel II (Q und Y bedeuten zusammen ein Sauerstoffatom), Halogenalkohole der Formel II (Q = OH, Y = Hal) oder Diole oder deren funktionelle Derivate der Formel II (Q = OH, Y = OH oder funktionell abgewandeltes OH) mit Aminen der Formel III ($Z = NH_2$) umsetzen; andererseits kann man Amine der Formel II (Q = OH, $Y = NH_2$) mit Verbindungen der Formel III (Z = X) zur Reaktion bringen. Die Umsetzung der genannten Epoxide mit Aminen der Formel $R{-}NH_2$ ist bevorzugt.

Die Umsetzung der Verbindungen der Formel II mit den Verbindungen der Formel III gelingt in Anwesenheit oder in Abwesenheit eines zusätzlichen inerten Lösungsmittels bei Temperaturen zwischen

etwa 0 und 150, vorzugsweise zwischen etwa 20 und 80°. Als inerte Lösungsmittel sind diejenigen geeignet, die für derartige Aminierungen aus der Literatur bekannt sind, beispielsweise Wasser, Alkohole wie Methanol, Aethanol, Isopropanol, n-Butanol; Aether wie Diäthyläther, Diisopropyläther, Tetrahydrofuran, Dioxan; Kohlenwasserstoffe wie Benzol, Toluol, Xylol; halogenierte Kohlenwasserstoffe wie Dichlormethan, Chloroform oder Trichloräthylen; Nitrile wie Acetonitril; Amide wie Dimethylformamid; Sulfoxide wie Dimethylsulfoxid. Es können auch Gemische dieser Lösungsmittel verwendet werden. Die Amine werden zweckmäßig mindestens im Molverhältnis 1 : 1 oder im Überschuß verwendet.

Es ist auch möglich, eine weitere Base zuzusetzen, z. B. eine anorganische Base wie Natrium- oder Kaliumhydroxid, Natriumcarbonat, Kaliumcarbonat, Natriumbicarbonat oder Kaliumbicarbonat. Geht man von Ausgangsverbindungen aus, die so konstituiert sind, daß bei der Umsetzung ein Mol Säure abgespalten wird (z. B. von Halohydrinen, so daß Halogenwasserstoff abgespalten wird), so ist es zweckmäßig, entweder eine zusätzliche Base oder einen Überschuß des Amins einzusetzen.

Falls X eine OH-Gruppe, aber auch eine Alkyl- oder Arylsulfonyloxygruppe bedeutet, kann sich auch der Zusatz eines sauren Katalysators empfehlen, z. B. einer anorganischen Säure (wie Schwefelsäure, Polyphosphorsäure, Bromwasserstoffsäure, Chlorwasserstoffsäure) und/oder einer organischen Säure (wie Ameisen-, Essig-, Propion- oder p-Toluolsulfonsäure). Ein Überschuß der Säure kann auch gleichzeitig als Lösungsmittel dienen.

Die erforderlichen Reaktionszeiten liegen zwischen etwa 10 Minuten und 7 Tagen, je nach den verwendeten Ausgangsstoffen und der Reaktionstemperatur. Man kann auch unter Druck (bis zu etwa 202,65 bar) arbeiten und so die Reaktion beschleunigen.

Die Verbindungen der Formel I können ferner durch Reaktion der Phenole der Formel IV mit den Nitratoalkylaminen der Formel V erhalten werden. Beispielsweise kann das Phenol IV zunächst in ein Salz übergeführt werden, insbesondere ein Metallsalz, z. B. ein Alkalimetallsalz (Li-, Na- oder K-Salz). Man kann das Phenol mit einem Metallsalz bildenden Reagenz umsetzen, z. B. einem Alkalimetall (z. B. Na), einem Alkalimetallhydrid oder -amid (z. B. LiH oder NaH, $NaNH_2$ oder $KNH_2$), einem Alkalimetallalkoholat (worin der Alkohol-Teil vorzugsweise 1–4 C-Atome besitzt, z. B. Lithium-, Natrium- oder Kaliummethylat, -äthylat oder -tert.-butylat), einer Organometallverbindung (z. B. Butyllithium, Phenyllithium oder Phenylnatrium), einem Metallhydroxid, -carbonat oder -bicarbonat (z. B. des Li, Na, K oder Ca). Die Herstellung des Phenolats wird vorteilhafterweise in Gegenwart eines Lösungsmittels oder Lösungsmittelgemischs vorgenommen. Geeignete Lösungsmittel sind z. B. Kohlenwasserstoffe (wie Hexan, Benzol, Toluol oder Xylol), Aether (z. B. Diäthyläther, Diisopropyläther, Tetrahydrofuran, Dioxan oder Diäthylenglykol-dimethyläther), Amide (z. B. Dimethylformamid), Alkohole (z. B. Methanol oder Aethanol), Ketone (z. B. Aceton oder Butanon).

Das Phenol IV oder dessen Salz wird mit der Verbindung V vorzugsweise in Gegenwart eines Verdünnungsmittels umgesetzt, z. B. desjenigen Lösungsmittels, das für die Herstellung des Salzes verwendet worden ist, das jedoch durch ein anderes Lösungsmittel ersetzt oder mit einem solchen vedünnt sein kann. Die Umsetzung wird in der Regel bei Temperaturen zwischen etwa −20 und 150° durchgeführt, vorzugsweise zwischen 20 und 80°.

Das Phenolat kann auch in situ gebildet werden. In diesem Falle läßt man das Phenol IV und die Verbindung V miteinander in Gegenwart einer Base reagieren. Eine besonders bevorzugte Methode besteht darin, daß man die Verbindungen IV und V zusammen mit einer alkoholisch-wässerigen Natriumhydroxidlösung etwa 5 bis 15 Stunden erhitzt.

Die Phenoxy-amino-propanole der Formel I sind ferner erhältlich durch Solvolyse einer Verbindung, die der Formel I entspricht, die jedoch zusätzlich anstelle eines oder mehrerer H-Atome eine oder mehrere solvolytisch abspaltbare Gruppe(n) trägt.

Als Ausgangsstoffe für diese Verfahrensvariante eignen sich insbesondere Verbindungen der allgemeinen Formel

$$Ar-O-CH_2-CHOR^3-CH_2-NR-R^4 \qquad VII$$

worin der Rest $R^3$ H oder eine Hydroxyschutzgruppe und der Rest $R^4$ H oder eine Aminoschutzgruppe bedeuten, die Reste $R^3$ und $R^4$ jedoch nicht gleichzeitig H bedeuten können und Ar und R die oben angegebenen Bedeutungen haben.

Die Ausdrücke »Hydroxyschutzgruppe« und »Aminoschutzgruppe« sind allgemein bekannt und beziehen sich auf Gruppen, die geeignet sind, eine Hydroxygruppe oder eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Da diese Schutzgruppen nach der erfindungsgemäßen Verfahrensvariante entfernt werden, ist ihre Art und Größe im übrigen nicht kritisch. Bevorzugt bedeuten $R^3$ und/oder $R^4$ jedoch Acyl mit 1–20, insbesondere 1–8 C-Atomen (z. B. Alkanoyl wie Acetyl, Aroyl wie Benzoyl, Aralkanoyl wie Phenylacetyl, Alkoxycarbonyl wie Methoxycarbonyl, Aralkyloxycarbonyl wie Benzyloxycarbonyl, Arylsulfonyl wie p-Toluolsulfonyl) oder gegebenenfalls substituiertes Benzyl (z. B. Benzyl, p-Nitrobenzyl, Triphenylmethyl).

Eine Solvolyse dieser Verbindungen gelingt zweckmäßig durch Einwirkung eines Lösungsmittels

wie Wasser (Hydrolyse) oder eines Alkohols mit vorzugsweise 1–4 C-Atomen (Alkoholyse) in Gegenwart eines sauren oder basischen Katalysators, z. B. einer Mineralsäure wie Schwefelsäure oder Salzsäure, eines Metallhydroxids wie Natrium-, Kalium-, Calcium-, Barium-, Blei- oder Silberhydroxid, oder eines Metall- oder Ammoniumsalzes wie Natrium- oder Kaliumcarbonat oder Ammoniumchlorid. Als Alkohole dienen vorzugsweise Methanol, Aethanol oder Isopropanol; man kann auch Gemische von Wasser mit einem dieser Alkohole verwenden. Die Solvolyse erfolgt zweckmäßig bei Temperaturen zwischen etwa 0 und etwa 80°.

Die Nitratester der Formel I sind weiterhin erhältlich durch partielle Veresterung entsprechender Diole der Formel VI oder ihrer reaktionsfähigen Derivate (z. B. ihrer Chlor-, Brom- oder Jodderivate; entsprechend VI, aber Chlor-, Brom- oder Jodalkyl an Stelle von E) mit Salpetersäure oder einem ihrer reaktionsfähigen Derivate, z. B. einem ihrer Salze, insbesondere dem Silber- oder Quecksilber(I)-salz. Die Veresterung wird beispielsweise durchgeführt in Gegenwart eines inerten Lösungsmittels, z. B. eines Äthers wie Tetrahydrofuran oder 1,2-Dimethoxyäthan oder in Gegenwart von Acetanhydrid, bei Temperaturen zwischen etwa 20 und 80°.

Eine Base der Formel I kann mit einer Säure in das zugehörige Säureadditionssalz übergeführt werden. Für diese Umsetzung kommen Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, ferner organische Säuren, insbesondere aliphatische, alicyclische, araliphatische, aromatische oder heterocyclische ein- oder mehrbasige Carbon- oder Sulfonsäuren, wie Ameisensäure, Essigsäure, Propionsäure, Pivalinsäure, Diäthylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenyl-propionsäure, Citronensäure, Gluconsäure, Ascorbinsäure, Nicotinsäure, Isonicotinsäure, Methan- oder Äthansulfonsäure, Äthandisulfonsäure, 2-Hydroxyäthansulfonsäure, Benzolsulfonsäure, p-Toluolsulfonsäure, Naphthalin-mono- und -disulfonsäuren. Die freien Basen der Formel I können, falls gewünscht, aus ihren Salzen durch Behandlung mit starken Basen, wie Natrium- oder Kaliumhydroxid, Natrium- oder Kaliumcarbonat, in Freiheit gesetzt werden.

Die Verbindungen der Formel I liegen gewöhnlich in racemischer Form vor. Weisen die Verbindungen zwei oder mehrere Asymmetriezentren auf, dann fallen sie bei der Synthese im allgemeinen als Gemische von Racematen an, aus denen man die einzelnen Racemate beispielsweise durch mehrmaliges Umkristallisieren aus geeigneten Lösungsmitteln isolieren und in reiner Form gewinnen kann.

Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch aktiven Trennmittel Diastereomere gebildet. Als Trennmittel eignen sich z. B. optisch aktive Säuren, wie die D- und L-Formen von Weinsäure, Dibenzoylweinsäure, Diacetylweinsäure, $\beta$-Camphersulfonsäure, Mandelsäure, Äpfelsäure oder Milchsäure.

Weiterhin ist es möglich, optisch aktive Verbindungen nach den oben beschriebenen Methoden zu erhalten, indem man Ausgangsstoffe verwendet, die bereits optisch aktiv sind.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen oder halbflüssigen Träger- oder Hilfsstoff und, falls gewünscht, in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Stoffe in Frage, die sich für die enterale (z. B. orale), parenterale oder topikale Applikation eignen und mit den neuen Verbindungen nicht reagieren, beispielsweise Wasser, pflanzliche Öle, Benzylalkohole, Polyäthylenglykole, Glycerintriacetat, Gelatine, Kohlehydrate wie Lactose oder Stärke, Magnessiumstearat, Talk, Vaseline (ein eingetragenes Warenzeichen). Zur oralen Applikation dienen insbesondere Tabletten, Dragees, Kapseln, Sirupe, Säfte oder Tropfen, zur rektalen Anwendung Suppositorien, zur parenteralen Applikation Lösungen, vorzugsweise ölige oder wässerige Lösungen, ferner Suspensionen, Emulsionen oder Implantate, für die topikale Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Gegenstand der Erfindung sind ferner die Verbindungen der Formel I zur Bekämpfung von Krankheiten, insbesondere von Herz-, Kreislauf- und Gefäßerkrankungen.

Dabei werden die erfindungsgemäßen Substanzen in der Regel in Analogie zu bekannten, im Handel befindlichen Herz- und Kreislaufmitteln, insbesondere $\beta$-Rezeptorenblockern, verabreicht, vorzugsweise in Dosierungen zwischen etwa 0,5 und 100 mg, insbesondere zwischen 2 und 50 mg pro

Dosierungseinheit. Die tägliche Dosierung liegt vorzugsweise zwischen etwa 0,01 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Jede der in den folgenden Beispielen genannten Verbindungen der Formel I ist zur Herstellung von pharmazeutischen Zubereitungen besonders geeignet.

In den nachfolgenden Beispielen bedeutet »übliche Aufarbeitung«: Man gibt, falls erforderlich, Wasser hinzu, extrahiert mit einem organischen Lösungsmittel wie Äthylacetat, Chloroform oder Dichlormethan, trennt ab, trocknet die organische Phase über Natriumsulfat, filtriert, dampft ein und reinigt durch Chromatographie und/oder Kristallisation (der Base oder eines ihrer Salze). Temperaturen sind in °C angegeben.

## Beispiel 1

Man löst 21,3 g 1,1-Dimethyl-3-nitratopropylaminnitrat in 200 ml Methanol, versetzt unter Rühren bei 20° mit 5,4 g Na-Methylat, darauf mit 24,3 g 1-Chlor-3-o-allyloxyphenoxy-2-propanol und erwärmt das Gemisch 18 Stunden auf 50°. Man kühlt ab, arbeitet wie üblich auf und erhält 1-o-Allyloxyphenoxy-3-(1,1-dimethyl-3-nitratopropylamino)-2-propanol. Halbfumarat, F. 98–99°.

## Beispiele 2 bis 146

Analog Beispiel 1 erhält man aus den entsprechenden 1-Chlor-3-ArO-2-propanolen oder 1,2-Epoxy-3-ArO-propanen mit den entsprechenden Nitratoalkylaminen:

2. 1-o-Isopropylphenoxy-3-(2-nitratoäthylamino)-2-propanol, Halbfumarat, F. 122–123°.
3. 1-o-Allylphenoxy-3-(2-nitratoäthylamino)-2-propanol, Halbfumarat, F. 131–133°.
4. 1-(1-Naphthyloxy)-3-(2-nitratoäthylamino)-2-propanol, Halbfumarat, F. 117–120°.
5. 1-Phenoxy-3-(1-methyl-3-nitratopropylamino)-2-propanol.
6. 1-o-Chlorphenoxy-3-(1-methyl-3-nitratopropylamino)-2-propanol.
7. 1-(2,5-Dichlorphenoxy)-3-(1-methyl-3-nitratopropylamino)-2-propanol.
8. 1-(2-Methyl-5-chlorphenoxy)-3-(1-methyl-3-nitratopropylamino)-2-propanol.
9. 1-o-Cyanphenoxy-3-(1-methyl-3-nitratopropylamino)-2-propanol, Halbfumarat, F. 131–133°.
10. 1-p-Cyanphenoxy-3-(1-methyl-3-nitratopropylamino)-1-propanol.
11. 1-p-Formylaminophenoxy-3-(1-methyl-3-nitratopropylamino)-2-propanol.
12. 1-o-Acetylaminophenoxy-3-(1-methyl-3-nitratopropylamino)-2-propanol.
13. 1-p-Acetylaminophenoxy-3-(1-methyl-3-nitratopropylamino)-2-propanol, Halbfumarat, F. 140–142°.
14. 1-p-Heptanoylaminophenoxy-3-(1-methyl-3-nitratopropylamino)-2-propanol.
15. 1-o-Formylphenoxy-3-(1-methyl-3-nitratopropylamino)-2-propanol.
16. 1-o-Acetylphenoxy-3-(1-methyl-3-nitratopropylamino)-2-propanol.
17. 1-o-Butyrylphenoxy-3-(1-methyl-3-nitratopropylamino)-2-propanol.
18. 1-o-Tolyloxy-3-(1-methyl-3-nitratopropylamino)-2-propanol.
19. 1-m-Tolyloxy-3-(1-methyl-3-nitratopropylamino)-2-propanol.
20. 1-p-Tolyloxy-3-(1-methyl-3-nitratopropylamino)-2-propanol.
21. 1-o-Aethylphenoxy-3-(1-methyl-3-nitratopropylamino)-2-propanol.
22. 1-o-Isopropylphenoxy-3-(1-methyl-3-nitratopropylamino)-2-propanol, Halbfumarat, F. 130–132°.
23. 1-p-Isopropylphenoxy-3-(1-methyl-3-nitratopropylamino)-2-propanol.
24. 1-(2,3-Dimethylphenoxy)-3-(1-methyl-3-nitratopropylamino)-2-propanol, Halbfumarat, F. 152°.
25. 1-o-Methoxyphenoxy-3-(1-methyl-3-nitratopropylamino)-2-propanol, Halbfumarat, F. 127–128°.
26. 1-p-Methoxyphenoxy-3-(1-methyl-3-nitratopropylamino)-2-propanol.
27. 1-o-Äthoxyphenoxy-3-(1-methyl-3-nitratopropylamino)-2-propanol.
28. 1-(p-2-Methoxyäthylphenoxy)-3-(1-methyl-3-nitratopropylamino)-2-propanol.
29. 1-(p-2-Isopropoxyäthoxymethylphenoxy)-3-(1-methyl-3-nitratopropylamino)-2-propanol, Halbfumarat, F. 90–92°.
30. 1-o-Methylthiophenoxy-3-(1-methyl-3-nitratopropylamino)-2-propanol.
31. 1-p-Methylthiophenoxy-3-(1-methyl-3-nitratopropylamino)-2-propanol.
32. 1-o-Vinylphenoxy-3-(1-methyl-3-nitratopropylamino)-2-propanol.
33. 1-o-Allylphenoxy-3-(1-methyl-3-nitratopropylamino)-2-propanol, Halbfumarat, F. 121–124°.
34. 1-p-Allylphenoxy-3-(1-methyl-3-nitratopropylamino)-2-propanol.

35. 1-o-Allyloxyphenoxy-3-(1-methyl-3-nitratopropylamino)-2-propanol, Halbfumarat, F. 100–102°.
36. 1-p-Allyloxyphenoxy-3-(1-methyl-3-nitratopropylamino)-2-propanol.
37. 1-o-Propargylphenoxy-3-(1-methyl-3-nitratopropylamino)-2-propanol.
38. 1-p-Propargylphenoxy-3-(1-methyl-3-nitratopropylamino)-2-propanol.
39. 1-o-Propargyloxyphenoxy-3-(1-methyl-3-nitratopropylamino)-2-propanol.
40. 1-p-Propargyloxyphenoxy-3-(1-methyl-3-nitratopropylamino)-2-propanol.
41. 1-o-Cyclopropylphenoxy-3-(1-methyl-3-nitratopropylamino)-2-propanol.
42. 1-o-Cyclobutylphenoxy-3-(1-methyl-3-nitratopropylamino)-2-propanol.
43. 1-o-Cyclopentylphenoxy-3-(1-methyl-3-nitratopropylamino)-2-propanol.
44. 1-o-Cyclohexylphenoxy-3-(1-methyl-3-nitratopropylamino)-2-propanol.
45. 1-o-Cycloheptylphenoxy-3-(1-methyl-3-nitratopropylamino)-2-propanol.
46. 1-o-Cyclooctylphenoxy-3-(1-methyl-3-nitratopropylamino)-2-propanol.
47. 1-o-Cyclopropyloxyphenoxy-3-(1-methyl-3-nitratopropylamino)-2-propanol.
48. 1-p-Cyclopropyloxyphenoxy-3-(1-methyl-3-nitratopropylamino)-2-propanol.
49. 1-o-Cyclobutyloxyphenoxy-3-(1-methyl-3-nitratopropylamino)-2-propanol.
50. 1-p-Cyclobutyloxyphenoxy-3-(1-methyl-3-nitratopropylamino)-2-propanol.
51. 1-o-Cyclopentyloxyphenoxy-3-(1-methyl-3-nitratopropylamino)-2-propanol.
52. 1-p-Cyclopentyloxyphenoxy-3-(1-methyl-3-nitratopropylamino)-2-propanol.
53. 1-o-Cyclohexyloxyphenoxy-3-(1-methyl-3-nitratopropylamino)-2-propanol.
54. 1-p-Cyclohexyloxyphenoxy-3-(1-methyl-3-nitratopropylamino)-2-propanol.
55. 1-o-Cycloheptyloxyphenoxy-3-(1-methyl-3-nitratopropylamino)-2-propanol.
56. 1-o-Cyclooctyloxyphenoxy-3-(1-methyl-3-nitratopropylamino)-2-propanol.
57. 1-(1-Naphthyloxy)-3-(1-methyl-3-nitratopropylamino)-2-propanol, Halbfumarat, F. 155–157°.
58. 1-(4-Indanyloxy)-3-(1-methyl-3-nitratopropylamino)-2-propanol.
59. 1-(4-Indenyloxy)-3-(1-methyl-3-nitratopropylamino)-2-propanol.
60. 1-(5-Tetralyloxy)-3-(1-methyl-3-nitratopropylamino)-2-propanol.
61. 1-(4-Indolyloxy)-3-(1-methyl-3-nitratopropylamino)-2-propanol, F 57–60°.
62. 1-(2-Methyl-4-indolyloxy)-3-(1-methyl-3-nitratopropylamino)-2-propanol.
63. 1-(2,3-Dimethyl-4-indolyloxy)-3-(1-methyl-3-nitratopropylamino)-2-propanol.
64. 1-(4-Carbazolyloxy)-3-(1-methyl-3-nitratopropylamino)-2-propanol, F. 111–113°.
65. 1-(1,2,3,4-Tetrahydro-2-oxo-5-chinolyloxy)-3-(1-methyl-3-nitratopropylamino)-2-propanol.
66. 1-Phenoxy-3-(1,1-dimethyl-3-nitratopropylamino)-2-propanol, Halbfumarat, F. 128°.
67. 1-o-Chlorphenoxy-3-(1,1-dimethyl-3-nitratopropylamino)-2-propanol.
68. 1-(2,5-Dichlorphenoxy)-3-(1,1-dimethyl-3-nitratopropylamino)-2-propanol.
69. 1-(2-Methyl-5-chlorphenoxy)-3-(1,1-dimethyl-3-nitratopropylamino)-2-propanol, Halbfumarat, F. 131–132°.
70. 1-o-Cyanphenoxy-3-(1,1-dimethyl-3-nitratopropylamino)-2-propanol, Halbfumarat, F. 91–94°.
71. 1-p-Cyanphenoxy-3-(1,1-dimethyl-3-nitratopropylamino)-2-propanol.
72. 1-p-Formylaminophenoxy-3-(1,1-dimethyl-3-nitratopropylamino)-2-propanol.
73. 1-o-Acetylaminophenoxy-3-(1,1-dimethyl-3-nitratopropylamino)-2-propanol.
74. 1-p-Acetylaminophenoxy-3-(1,1-dimethyl-3-nitratopropylamino)-2-propanol.
75. 1-p-Heptanoylaminophenoxy-3-(1,1-dimethyl-3-nitratopropylamino)-2-propanol.
76. 1-o-Formylphenoxy-3-(1,1-dimethyl-3-nitratopropylamino)-2-propanol.
77. 1-o-Acetylphenoxy-3-(1,1-dimethyl-3-nitratopropylamino)-2-propanol.
78. 1-o-Butyrylphenoxy-3-(1,1-dimethyl-3-nitratopropylamino)-2-propanol.
79. 1-o-Tolyloxy-3-(1,1-dimethyl-3-nitratopropylamino)-2-propanol.
80. 1-m-Tolyloxy-3-(1,1-dimethyl-3-nitratopropylamino)-2-propanol.
81. 1-p-Tolyloxy-3-(1,1-dimethyl-3-nitratopropylamino)-2-propanol.
82. 1-o-Aethylphenoxy-3-(1,1-dimethyl-3-nitratopropylamino)-2-propanol.
83. 1-o-Isopropylphenoxy-3-(1,1-dimethyl-3-nitratopropylamino)-2-propanol, Halbfumarat, F. 128°.
84. 1-p-Isopropylphenoxy-3-(1,1-dimethyl-3-nitratopropylamino)-2-propanol.
85. 1-(2,3-Dimethylphenoxy)-3-(1,1-dimethyl-3-nitratopropylamino)-2-propanol.
86. 1-o-Methoxyphenoxy-3-(1,1-dimethyl-3-nitratopropylamino)-2-propanol, Halbfumarat, F. 119–121°.
87. 1-p-Methoxyphenoxy-3-(1,1-dimethyl-3-nitratopropylamino)-2-propanol.
88. 1-o-Aethoxyphenoxy-3-(1,1-dimethyl-3-nitratopropylamino)-2-propanol.
89. 1-(p-2-Methoxyäthylphenoxy)-3-(1,1-dimethyl-3-nitratopropylamino)-2-propanol.
90. 1-(p-2-Isopropoxyäthoxymethylphenoxy)-3-(1,1-dimethyl-3-nitratopropylamino)-2-propanol.
91. 1-o-Methylthiophenoxy-3-(1,1-dimethyl-3-nitratopropylamino)-2-propanol.
92. 1-p-Methylthiophenoxy-3-(1,1-dimethyl-3-nitratopropylamino)-2-propanol.
93. 1-o-Vinylphenoxy-3-(1,1-dimethyl-3-nitratopropylamino)-2-propanol.
94. 1-o-Allylphenoxy-3-(1,1-dimethyl-3-nitratopropylamino)-2-propanol.
95. 1-p-Allylphenoxy-3-(1,1-dimethyl-3-nitratopropylamino)-2-propanol.

96. 1-p-Allyloxyphenoxy-3-(1,1-dimethyl-3-nitratopropylamino)-2-propanol.
97. 1-o-Propargylphenoxy-3-(1,1-dimethyl-3-nitratopropylamino)-2-propanol.
98. 1-p-Propargylphenoxy-3-(1,1-dimethyl-3-nitratopropylamino)-2-propanol.
99. 1-o-Propargyloxyphenoxy-3-(1,1-dimethyl-3-nitratopropylamino)-2-propanol.
100. 1-p-Propargyloxyphenoxy-3-(1,1-dimethyl-3-nitratopropylamino)-2-propanol.
101. 1-o-Cyclopropylphenoxy-3-(1,1-dimethyl-3-nitratopropylamino)-2-propanol.
102. 1-o-Cyclobutylphenoxy-3-(1,1-dimethyl-3-nitratopropylamino)-2-propanol.
103. 1-o-Cyclopentylphenoxy-3-(1,1-dimethyl-3-nitratopropylamino)-2-propanol.
104. 1-o-Cyclohexylphenoxy-3-(1,1-dimethyl-3-nitratopropylamino)-2-propanol, Halbfumarat, F. 125–127°.
105. 1-o-Cycloheptylphenoxy-3-(1,1-dimethyl-3-nitratopropylamino)-2-propanol.
106. 1-o-Cyclooctylphenoxy-3-(1,1-dimethyl-3-nitratopropylamino)-2-propanol.
107. 1-o-Cyclopropyloxyphenoxy-3-(1,1-dimethyl-3-nitratopropylamino)-2-propanol.
108. 1-p-Cyclopropyloxyphenoxy-3-(1,1-dimethyl-3-nitratopropylamino)-2-propanol.
109. 1-o-Cyclobutyloxyphenoxy-3-(1,1-dimethyl-3-nitratopropylamino)-2-propanol.
110. 1-p-Cyclobutyloxyphenoxy-3-(1,1-dimethyl-3-nitratopropylamino)-2-propanol.
111. 1-o-Cyclopentyloxyphenoxy-3-(1,1-dimethyl-3-nitratopropylamino)-2-propanol.
112. 1-p-Cyclopentyloxyphenoxy-3-(1,1-dimethyl-3-nitratopropylamino)-2-propanol.
113. 1-o-Cyclohexyloxyphenoxy-3-(1,1-dimethyl-3-nitratopropylamino)-2-propanol.
114. 1-p-Cyclohexyloxyphenoxy-3-(1,1-dimethyl-3-nitratopropylamino)-2-propanol.
115. 1-o-Cycloheptyloxyphenoxy-3-(1,1-dimethyl-3-nitratopropylamino)-2-propanol.
116. 1-o-Cyclooctyloxyphenoxy-3-(1,1-dimethyl-3-nitratopropylamino)-2-propanol.
117. 1-(1-Naphthyloxy)-3-(1,1-dimethyl-3-nitratopropylamino)-2-propanol.
118. 1-(4-Indanyloxy)-3-(1,1-dimethyl-3-nitratopropylamino)-2-propanol.
119. 1-(4-Indenyloxy)-3-(1,1-dimethyl-3-nitratopropylamino)-2-propanol.
120. 1-(5-Tetralyloxy)-3-(1,1-dimethyl-3-nitratopropylamino)-2-propanol.
121. 1-(4-Indolyloxy)-3-(1,1-dimethyl-3-nitratopropylamino)-2-propanol.
122. 1-(2-Methyl-4-indolyloxy)-3-(1,1-dimethyl-3-nitratopropylamino)-2-propanol.
123. 1-(2,3-Dimethyl-4-indolyloxy)-3-(1,1-dimethyl-3-nitratopropylamino)-2-propanol.
124. 1-(4-Carbazolyloxy)-3-(1,1-dimethyl-3-nitratopropylamino)-2-propanol, F. 168–170°.
125. 1-(1,2,3,4-Tetrahydro-2-oxo-5-chinolyloxy)-3-(1,1-dimethyl-3-nitratopropylamino)-2-propanol.
126. 1-o-Cyanphenoxy-3-(1,1,3-trimethyl-3-nitratopropylamino)-2-propanol, Halbfumarat, F. 140–142°.
127. 1-o-Isopropylphenoxy-3-(1,1,3-trimethyl-3-nitratopropylamino)-2-propanol.
128. 1-o-Methoxyphenoxy-3-(1,1,3-trimethyl-3-nitratopropylamino)-2-propanol, Halbfumarat, F. 122–123°.
129. 1-o-Allyloxyphenoxy-3-(1,1,3-trimethyl-3-nitratopropylamino)-2-propanol, Halbfumarat, F. 97–99°.
130. 1-(4-Indenyloxy)-3-(1,1,3-trimethyl-3-nitratopropylamino)-2-propanol.
131. 1-(4-Indolyloxy)-3-(1,1,3-trimethyl-3-nitratopropylamino)-2-propanol.
132. 1-(4-Carbazolyloxy)-3-(1,1,3-trimethyl-3-nitratopropylamino)-2-propanol.
133. 1-o-Cyanphenoxy-3-(1-methyl-4-nitratobutylamino)-2-propanol.
134. 1-o-Isopropylphenoxy-3-(1-methyl-4-nitratobutylamino)-2-propanol.
135. 1-o-Methoxyphenoxy-3-(1-methyl-4-nitratobutylamino)-2-propanol.
136. 1-o-Allyloxyphenoxy-3-(1-methyl-4-nitratobutylamino)-2-propanol.
137. 1-(4-Indenyloxy)-3-(1-methyl-4-nitratobutylamino)-2-propanol.
138. 1-(4-Indolyloxy)-3-(1-methyl-4-nitratobutylamino)-2-propanol.
139. 1-(4-Carbazolyloxy)-3-(1-methyl-4-nitratobutylamino)-2-propanol.
140. 1-o-Cyanphenoxy-3-(1,1-dimethyl-4-nitratobutylamino)-2-propanol.
141. 1-o-Isopropylphenoxy-3-(1,1-dimethyl-4-nitratobutylamino)-2-propanol.
142. 1-o-Methoxyphenoxy-3-(1,1-dimethyl-4-nitratobutylamino)-2-propanol.
143. 1-o-Allyloxyphenoxy-3-(1,1-dimethyl-4-nitratobutylamino)-2-propanol.
144. 1-(4-Indenyloxy)-3-(1,1-dimethyl-4-nitratobutylamino)-2-propanol.
145. 1-(4-Indolyloxy)-3-(1,1-dimethyl-4-nitratobutylamino)-2-propanol.
146. 1-(4-Carbazolyloxy)-3-(1,1-dimethyl-4-nitratobutylamino)-2-propanol.

## Beispiel 147

Ein Gemisch von 17,5 g 1-o-Cyanphenoxy-2,3-epoxypropan und 14,8 g 1,1-Dimethyl-3-nitratopropylamin in 45 ml Aethanol wird 15 Stunden bei 20° stehengelassen. Man dampft ein, arbeitet wie üblich auf und erhält 1-o-Cyanphenoxy-3-(1,1-dimethyl-3-nitratopropylamino)-2-propanol, F. 91–94°.

Beispiel 148

Eine Lösung von 20,6 g 1-o-Allyloxyphenoxy-2,3-epoxypropan und 15 g 1-Methyl-3-nitratopropylamin in 200 ml Methanol wird 60 Stunden bei 25° gerührt und anschließend eingedampft. Nach üblicher Aufarbeitung erhält man 1-o-Allyloxyphenoxy-3-(1-methyl-3-nitratopropylamino)-2-propanol. Halbfumarat, F. 100–102°.

Beispiel 149

Ein Gemisch aus 19,7 g 1-o-Methoxyphenoxy-3-amino-2-propanol [erhältlich durch Umsetzung von 1-o-Methoxyphenoxy-2,3-epoxypropan mit $NH_3$], 13,8 g Kaliumcarbonat, 22 g 1-Methyl-3-nitratopropylbromid und 100 ml Tetrahydrofuran wird unter Rühren 24 Stunden gekocht. Man saugt ab, dampft das Filtrat ein, arbeitet wie üblich auf und erhält 1-o-Methoxyphenoxy-3-(1-methyl-3-nitratopropylamino)-2-propanol, Halbfumarat, F. 127–128°.

Beispiel 150

Ein Gemisch aus 13,3 g 4-Hydroxyindol, 22,7 g 1-Chlor-3-(1-methyl-3-nitratopropylamino)-2-propanol (erhältlich aus Epichlorhydrin und 1-Methyl-3-nitratopropylamin), 8 g Natriumhydroxid, 400 ml Äthanol und 20 ml Wasser wird 10 Stunden lang auf 70° erhitzt. Man dampft ein, arbeitet wie üblich auf und erhält 1-(4-Indolyloxy)-3-(1-methyl-3-nitratopropylamino)-2-propanol, F. 57–60°.

Beispiel 151

Man erwärmt 10 g N-[2-Hydroxy-3-(p-2-isopropoxyäthoxymethylphenoxy)-propyl]-N-(1-methyl-3-nitratopropyl)-acetamid [erhältlich durch Umsetzung von Na-p-(2-Isopropoxyäthoxymethyl)-phenolat mit N-(2-Hydroxy-3-brom-propyl-N-(1-methyl-3-nitratopropyl)-acetamid] mit 250 ml 20%iger Salzsäure 4 Stunden auf 50°, dampft ein, arbeitet wie üblich auf und erhält 1-(p-2-Isopropoxyäthoxymethylphenoxy)-3-(1-methyl-3-nitratopropylamino)-2-propanol. Halbfumarat, F. 90–92°.

Beispiel 152

Man erwärmt 10 g 1-o-Methoxyphenoxy-2-acetoxy-3-(1-methyl-3-nitratopropylamino)-propan [erhältlich aus Na-o-Methoxyphenolat und 1-Brom-2-acetoxy-3-(1-methyl-3-nitratopropylamino)-propan] mit 250 ml 10%iger äthanolischer NaOH 2 Stunden auf 50°, dampft ein, arbeitet wie üblich auf und erhält 1-o-Methoxyphenoxy-3-(1-methyl-3-nitratopropylamino)-2-propanol, Halbfumarat, F. 127–128°.

Beispiel 153

Ein Gemisch von 4,05 g 1-(4-Carbazolyloxy)-3-(1,1-dimethyl-3-brompropylamino)-2-propanol, 2,8 g Quecksilber(I)nitrat und 60 ml 1,2 Dimethoxyäthan wird 2 Stunden auf 50° erwärmt. Nach üblicher Aufarbeitung erhält man 1-(4-Carbazolyloxy)-3-(1,1-dimethyl-3-nitratopropylamino)-2-propanol, F. 168–170°.

Die nachstehenden Beispiele betreffen pharmazeutische Zubereitungen, die Amine der Formel I oder ihre Säureadditionssalze enthalten:

Beispiel A
Tabletten

Ein Gemisch von 1 kg 1-o-Allyloxy-phenoxy-3-(1-methyl-3-nitratopropylamino)-2-propanol-halbfumarat, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B
Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C
Kapseln

2 kg 1-o-Methoxy-phenoxy-3-(1,1-dimethyl-3-nitratopropylamino)-2-propanol-halbfumarat werden in üblicher Weise in Hartgelatinekapseln gefüllt, so daß jede Kapsel 20 mg des Wirkstoffs enthält.

Beispiel D
Ampullen

Eine Lösung von 1 kg 1-(4-Indolyloxy)-3-(1-methyl-3-nitratopropylamino)-2-propanol in 30 l zweifach destilliertem Wasser wird steril filtriert, in Ampullen abgefüllt, unter sterilen Bedingungen lyophilisiert und steril verschlossen. Jede Ampulle enthält 1 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Patentansprüche**

1. 1-Aryloxy-3-nitratoalkylamino-2-propanole der allgemeinen Formel

$$Ar{-}O{-}CH_2{-}CHOH{-}CH_2{-}NH{-}R \qquad I$$

worin

Ar Phenyl; ein- oder mehrfach durch Halogen, Cyan, Acylamino (worin die Acylgruppe 1–7 C-Atome besitzt), Alkanoyl mit 1–4 C-Atomen, Alkyl (worin die Kette durch 1 oder 2 O-Atome oder durch ein S-Atom unterbrochen und/oder worin eine Mehrfachbindung vorhanden sein kann) mit insgesamt 1–10 C-Atomen, Cycloalkyl mit 3–8 C-Atomen und/oder Cycloalkoxy mit 3–8 C-Atomen substituiertes Phenyl; Naphthyl; Indanyl; Indenyl; Tetralyl; Indolyl; ein- oder mehrfach durch Alkyl mit jeweils 1–4 C-Atomen substituiertes Indolyl; Carbazolyl; oder 1,2,3,4-Tetrahydro-2-oxo-chinolyl und

R Nitratoalkyl mit 2–10 C-Atomen bedeuten,

und ihre physiologisch unbedenklichen Säureadditionssalze.

2. a) 1-o-Allyloxy-phenoxy-3-(1-methyl-3-nitratopropylamino)-2-propanol.
b) 1-(1-Naphthyloxy)-3-(1-methyl-3-nitratopropylamino)-2-propanol.
c) 1-o-Isopropylphenoxy-3-(1-methyl-3-nitratopropylamino)-2-propanol.
d) 1-o-Methoxyphenoxy-3-(1-methyl-3-nitratopropylamino)-2-propanol.
e) 1-(4-Indolyloxy)-3-(1-methyl-3-nitratopropylamino)-2-propanol.
f) 1-o-Cyanphenoxy-3-(1,1-dimethyl-3-nitratopropylamino)-2-propanol.
g) 1-(4-Carbazolyloxy)-3-(1,1-dimethyl-3-nitratopropylamino)-2-propanol.

3. Verfahren zur Herstellung von 1-Aryloxy-3-nitratoalkylamino-2-propanolen der allgemeinen Formel I nach Patentanspruch 1 sowie von deren physiologisch unbedenklichen Säureadditionssalzen, dadurch gekennzeichnet, daß man eine Verbindung der allgemeinen Formel II

$$Ar{-}O{-}CH_2{-}CH_2Y \qquad II$$

mit einer Verbindung der allgemeinen Formel III

$$Z{-}R \qquad III$$

worin

der eine der Reste Y und Z $NH_2$,
der andere dieser Reste X,
Q OH oder zusammen mit X ein Sauerstoffatom,

| | |
|---|---|
| X | Hal, OH, eine funktionell abgewandelte OH-Gruppe oder (in II) zusammen mit Q ein Sauerstoffatom und |
| Hal | Cl, Br oder J bedeuten und |
| Ar und R | die oben angegebenen Bedeutungen nach Anspruch 1 haben, |

oder ein Phenol der allgemeinen Formel IV

$$Ar-OH \qquad IV$$

worin

Ar die oben angegebene Bedeutung hat

mit einem Nitratoalkylamin der allgemeinen Formel V

$$X-CH_2-CHQ-CH_2NHR \qquad V$$

worin

R, Q und X die oben angegebenen Bedeutungen haben

umsetzt,
oder daß man eine der allgemeinen Formel I entsprechende Verbindung, die jedoch zusätzlich an Stelle eines oder mehrerer H-Atome eine oder mehrere solvolytisch abspaltbare Gruppe(n) trägt, mit einem solvolysierenden Mittel behandelt,
oder daß man eine Verbindung der allgemeinen Formel VI

$$Ar-O-CH_2-CHOH-CH_2-NH-E \qquad VI$$

worin

E eine Hydroxyalkylgruppe mit 2–10 C-Atomen bedeutet

oder eines ihrer reaktionsfähigen Derivate mit Salpetersäure oder einem ihrer reaktionsfähigen Derivate verestert, und daß man gegebenenfalls eine erhaltene Base der Formel I durch Behandeln mit einer Säure in eines ihrer physiologisch unbedenklichen Säureadditionssalze umwandelt.

4. Verfahren zur Herstellung pharmazeutischer Zubereitungen, dadurch gekennzeichnet, daß man eine Verbindung der Formel I und/oder ihrer physiologisch unbedenklichen Säureadditionssalze zusammen mit mindestens einem festen, flüssigen oder halbflüssigen Träger- oder Hilfsstoff und gegebenenfalls zusammen mit einem weiteren Wirkstoff in eine geeignete Dosierungsform bringt.

5. Pharmazeutische Zubereitung, enthaltend eine Verbindung der allgemeinen Formel I und/oder eines ihrer physiologisch unbedenklichen Säureadditionssalze.

6. Verbindungen der allgemeinen Formel I nach Patentanspruch 1 zur Bekämpfung von Krankheiten.

**Claims**

1. 1-Aryloxy-3-nitratoalkylamino-2-propanols of the general formula I

$$Ar-O-CH_2-CHOH-CH_2-NH-R \qquad I$$

in which Ar is phenyl; phenyl monosubstituted or polysubstituted by halogen, cyano, acylamino (in which the acyl group has 1–7 C atoms), alkanoyl with 1–4 C atoms, alkyl (in which the chain can be interrupted by 1 or 2 O atoms or by one S atom and/or in which a multiple bond can be present) with a total of 1–10 C atoms, cycloalkyl with 3–8 C atoms and/or cycloalkoxy with 3–8 C atoms; naphthyl; indanyl; indenyl; tetralyl; indolyl; indolyl monosubstituted or polysubstituted by alkyl with, in each case, 1–4 C atoms; carbazolyl; or 1,2,3,4-tetrahydro-2-oxoquinolyl and R is nitratoalkyl with 2–10 C atoms, and their physiologically acceptable acid addition salts.

2. a) 1-o-Allyloxy-phenoxy-3-(1-methyl-3-nitratopropylamino)-2-propanol.
b) 1-(1-Naphthyloxy)-3-(1-methyl-3-nitratopropylamino)-2-propanol.
c) 1-o-Isopropylphenoxy-3-(1-methyl-3-nitratopropylamino)-2-propanol.
d) 1-o-Methoxyphenoxy-3-(1-methyl-3-nitratopropylamino)-2-propanol.
e) 1-(4-Indolyloxy)-3-(1-methyl-3-nitratopropylamino)-2-propanol.
f) 1-o-Cyanophenoxy-3-(1,1-dimethyl-3-nitratopropylamino)-2-propanol.
g) 1-(4-Carbazolyloxy)-3-(1,1-dimethyl-3-nitrato-propylamino)-2-propanol.

3. Process for the preparation of 1-aryloxy-3-nitrato-alkylamino-2-propanols of the general formula I according to patent claim 1 and of their physiologically acceptable acid addition salts, characterised in that a compound of the general formula II

$$Ar-O-CH_2-CHQ-CH_2Y \quad II$$

is reacted with a compound of the general formula III

$$Z-R \quad III$$

in which one of the radicals Y and Z is $NH_2$ an the other is X, Q is OH or together with X is an oxygen atom, X is Hal, OH, a functionally modified OH group or (in II) together with Q is an oxygen atom and Hal is Cl, Br or I and Ar and R are as defined above according to claim 1, or a phenol of the general formula IV

$$Ar-OH \quad IV$$

in which Ar is as defined above, is reacted with a nitratoalkylamine of the general formula V

$$X-CH_2CHQ-CH_2NHR \quad V$$

in which R, Q and X are as defined above, or in that a compound which corresponds to the general formula I but which additionally carries one or more solvolytically detachable groups in place of one or more H atoms is treated with a solvolysing agent, or in that a compound of the general formula VI

$$Ar-O-CH_2-CHOH-CH_2-NH-E \quad VI$$

in which E is a hydroxyalkyl group with 2–10 C atoms... (sic), or one of its reactive derivatives, is esterified with nitric acid or one of its reactive derivates, and in that, optionally, a resulting base of the formula I is converted by treatment with an acid into one of its physiologically acceptable acid addition salts.

4. Process for the preparation of pharmaceutical formulations, characterised in that a compound of the formula I and/or one of its physiologically acceptable acid addition salts is brought, together with at least one solid, liquid or semi-liquid excipient or auxiliary and, optionally, together with a further active ingredient, into a suitable dosage form.

5. Pharmaceutical formulation containing a compound of the general formula I and/or one of its physiologically acceptable acid addition salts.

6. Compounds of the general formula I according to patent claim 1 for combating diseases.

**Revendications**

1. 1-aryloxy-3-nitrato-alcoylamino-2-propanols de formule générale I:

$$Ar-O-CH_2CHOC-CH_2-NH-R \quad I$$

où Ar représente un phényle; un phényle mono- ou polysubstitué par un halogène, un cyano, un acylamino (où le groupe acyle possède de 1 à 7 atomes de carbone), un alcanoyle ayant de 1 à 4 atomes de carbone, un alcoyle ( où la chaîne peut être interrompue par un ou deux atomes d'oxygène ou par un atome de soufre et/ou une liaison multiple peut être présente) ayant au total de 1 à 10 atomes de carbone, un cycloalcoyle ayant de 3 à 8 atomes de carbone et/ou cycloalcoxy ayant de 3 à 8 atomes de carbone; un naphthyle; un indanyle; un indényle; un tétralyle; un indolyle; un indolyle mono- ou polysubstitué par un alcoyle en $C_1$ à $C_4$; un carbazolyle; ou un 1,2,3,4-tétrahydro-2-oxoquinoléyle et R représente un nitroalcoyle en $C_2$ à $C_{10}$, et leurs sels d'addition acides physiologiquement acceptables.

2. a) 1-o-Allyloxy-phénoxy-3-(1-méthyl-3-nitratopropylamino)-2-propanol.
b) 1-(1-Naphthyloxy)-3-(1-méthyl-3-nitratopropylamino)-2-propanol.
c) 1-o-Isopropylphénoxy-3-(1-méthyl-3-nitratopropylamino)-2-propanol.
d) 1-o-Méthoxyphénoxy-3-(1-méthyl-3-nitratopropylamino)-2-propanol.
e) 1-(4-Indolyloxy)-3-(1-méthyl-3-nitratopropylamino)-2-propanol.
f) 1-o-Cyanophénoxy-3-(1,1-diméthyl-3-nitratopropylamino)-2-propanol.
g) 1-(4-Carbazolyloxy)-3-(1,1-diméthyl-3-nitratopropylamino)-2-propanol.

3. Procédé de préparation de 1-aryloxy-3-nitratoalcoylamino-2-propanols de formule générale I selon la revendication 1 ainsi que de leurs sels d'addition acides physiologiquement acceptables,

caractérisé en ce qu'on fait réagir un composé de formule générale II:

$$Ar-O-CH_2CHQ-CH_2Y \qquad II$$

avec un composé de formule générale III:

$$Z-R \qquad III$$

où l'un des radicaux Y et Z représente $NH_2$, l'autre de ces radicaux représente X, Q représente OH ou, avec X, un atome d'oxygène, X représente Hal, OH, un groupe OH fonctionnellement modifié ou (dans II), avec Q, un atome d'oxygène, et Hal représente Cl, Br ou I et Ar et R ont les significations données ci-dessus, selon la revendication 1 ou un phénol de formule générale IV:

$$Ar-OH \qquad IV$$

où Ar à la signification donnée ci-dessus avec une nitratoalcoylamine de formule générale V:

$$X-CH_2CHQ-CH_2-NH-R \qquad V$$

où R, Q et X ont les significations données ci-dessus, ou en ce qu'on traite un composé correspondant à la formule générale I, qui porte cependant en outre à le place d'un ou plusieurs atome(s), d'hydrogène un ou plusieurs groupe(s) séparable(s) par solvolyse, avec un agent de solvolyse, ou en ce qu'on fait réagir un composé de formule générale VI:

$$Ar-O-CH_2CHOH-CH_2NH-E \qquad VI$$

où E représente un groupe hydroxyalcoyle ayant de 2 à 10 atomes de carbone, ou en ce qu'on estérifie, ou un de ses dérivés réactifs avec de l'acide nitrique ou en ce qu'on estérifie un de ses dérivés réactifs, et en ce qu'éventuellement on transforme une base de formule I obtenue par traitement avec un acide en un de ses sels d'addition acides physiologiquement acceptables.

4. Procédé de préparation de préparations pharmaceutiques, caractérisé en ce qu'on amène sous une forme posologique appropriée un composé de formule I et/ou un de ses sels d'addition acides physiologiquement acceptables avec au moins un support ou additif solide, liquide ou semi-liquide et éventuellement avec une autre substance active.

5. Préparation pharmaceutique contenant un composé de formule générale I et/ou un de ses sels d'addition acides physiologiquement acceptables.

6. Composés de formule générale I selon la revendication 1 pour la lutte contre les maladies.